Europäisches Patentamt

European Patent Office

Office européen des brevets

(11) Veröffentlichungsnummer: **0 385 212**
**A2**

(12) **EUROPÄISCHE PATENTANMELDUNG**

(21) Anmeldenummer: **90103128.6**

(51) Int. Cl.5: **A61M 15/00**

(22) Anmeldetag: **19.02.90**

(30) Priorität: **03.03.89 DE 8902559 U**

(43) Veröffentlichungstag der Anmeldung:
**05.09.90 Patentblatt 90/36**

(84) Benannte Vertragsstaaten:
**AT BE CH DE DK ES FR GB GR IT LI LU NL SE**

(71) Anmelder: **GAPLAST GMBH**
**Wurmansauer Strasse 22**
**D-8111 Altenau(DE)**

(72) Erfinder: **Kneer, Roland**
**Am Weide 11**
**D-8105 Farchant(DE)**

(74) Vertreter: **Huss, Carl-Hans, Dipl.-Ing.**
**Patentanwalt Griesstrasse 3 a Postfach 14 54**
**D-8100 Garmisch-Partenkirchen(DE)**

(54) **Inhalationsgefäss.**

(57) Die Erfindung richtet sich auf eine Inhalationshilfe in Form eines Gefäßes, das an eine Spraypatrone auf einer Seite angeschlossen werden kann, in dem sich der Inhalationsnebel entwickelt und aus dessen anderer Öffnung der Inhalationsnebel entnommen werden kann. Um für die Lagerung und den Transport ein bei Nichtgebrauch leicht verstaubares Inhalationsgefäß zu schaffen, das für den Gebrauch einfach in seine Gebrauchsgröße umgewandelt werden kann, schlägt die Erfindung bei einem solchen aus einem Anschlußstück (1), einem Entnahmestück (2) und einem zwischen diesen liegenden Gefäßkörper (3) bestehenden Gefäß vor, daß der Gefäßkörper aus ineinander verschiebbaren Ringen mit in Richtung auf das Anschlußstück (1) abnehmenden Durchmessern zusammengesetzt ist.

FIG.1

## Inhalationsgefäß

Die Erfindung richtet sich auf eine Inhalationshilfe in Form eines Gefäßes, das an eine im Handel erhältliche Spraypatrone auf einer Seite angeschlossen werden kann, in dem sich der Inhalationsnebel entwickelt und aus dessen anderer Öffnung der Inhalationsnebel dann entnommen wird.

Bekannte Gefäße oft beträchtlicher Größe sind aus einem Stück gefertigt und deshalb unhandlich, schlecht aufzubewahren und für die Mitnahme auf Urlaubs- oder Berufsreisen ungeeignet.

Demnach lag der Erfindung die Aufgabe zugrunde, ein für die Lagerung und den Transport bei Nichtgebrauch leicht verstaubares Inhalationsgefäß zu schaffen, das für den Gebrauch einfach in seine Gebrauchsgröße umgewandelt werden kann. Diese Aufgabe löst die Erfindung mit den in den Ansprüchen definierten Mitteln. Sie ist in der Zeichnung an einem, keinen Anspruch auf Vollständigkeit erhebenden Ausführungsbeispiel veranschaulicht und anhand desselben nachfolgend beschrieben. Es stellen dar:

Fig. 1 ein erfindungsgemäßes Gefäß in der Ansicht im Maßstab 2:1;

Fig. 2 einen Halbschnitt durch das Gefäß nach Fig. 1, geschnitten längs der Linie II-II in Fig. 1;

Fig. 3 eine Aufsicht auf das Gefäß nach Fig. 1, gesehen in Pfeilrichtung III in der Fig. 1;

Fig. 4 einen Schnitt durch ein Gefäß gemäß Fig. 2, jedoch in zusammengeschobenem Aufbewahrungszustand;

Fig. 5 eine Einzelheit in einem gegenüber den Fig. 1 bis 4 größeren Maßstab.

Das Gefäß nach der Erfindung besteht aus einem allgemein mit 1 bezeichneten Anschlußstück, dem allgemein mit 2 bezeichneten Entnahmestück und dem zwischen diesen Stücken liegenden, allgemein mit 3 bezeichneten Gefäßkörper.

Das Anschlußstück 1 besteht aus einem Stutzen 4, einem konischen Zwischenstück 5 und dem zylindrischen Ringteil 6. Der Stutzen 4 besitzt für das leichtere Einstecken der Mündung der Spraypatrone oder -dose eine sich zur Öffnung 7 hin erweiternde Konizität.

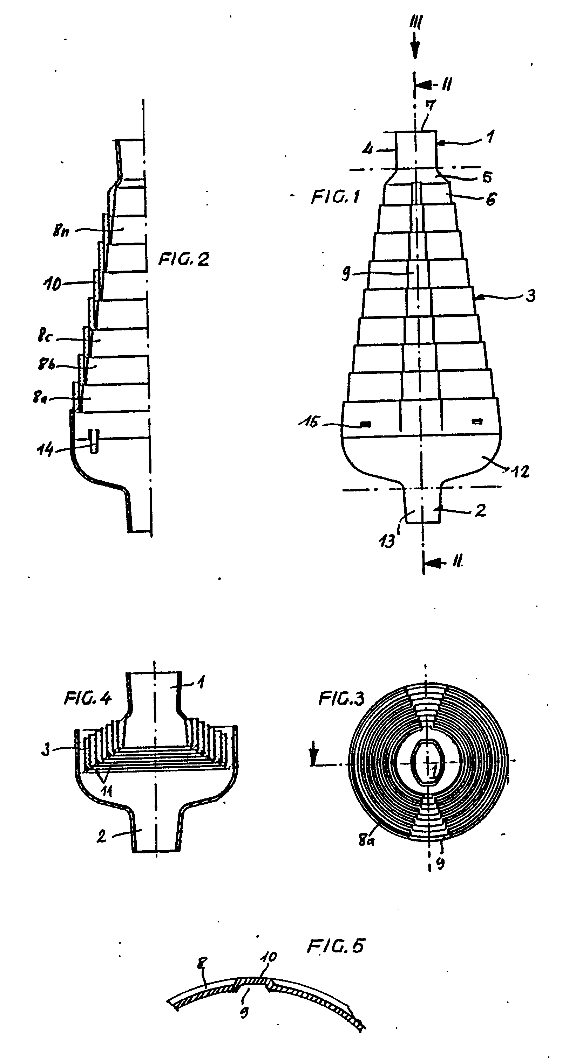

Der Gefäßkörper 3 ist aus zwischen Entnahmestück 2 und Anschlußstück 1 in den Durchmessern abnehmenden, ineinander verschiebbaren Ringen 8a, 8b, 8c ...8n zusammengesetzt. Jeder Ring weist eine zum Anschlußstück 1 hin abnehmende Konizität auf, wodurch erreicht wird, daß in dem in den Fig. 1 und 2 dargestellten, auseinandergezogenen Gebrauchszustand kein Ring aus dem darunter liegenden herausgezogen werden kann, der Körper also nicht auseinanderfallen und sich nicht in einzelne Ringe auflösen kann.

Jeder Ring besitzt ferner, wie insbesondere aus den Fig. 3 und 5 ersichtlich, auf einem Durchmesser liegend und der Konizität nicht folgend, Nuten 9 mit senkrechten Wänden 10. Diese werden gemäß Fig. 1 von unten nach oben schmäler, so daß die Nut des jeweils oben liegenden Ringes, z.B. des Ringes 8b, in die Nut des darunter liegenden Ringes, z.B. des Ringes 8a, eingreifen kann wodurch eine Verdrehung der Ringe gegeneinander verhindert wird. Natürlich können, um die Stabilität noch zu erhöhen, auch drei oder mehr Nuten 9 über den Umfang verteilt vorgesehen sein.

Außerdem weist jeder Ring einen unteren Bund 11 auf, der verhindert, daß die Ringe beim Zusammenschieben oder in zusammengeschobenem Zustand gemäß Fig 4 durchgedrückt werden können, also ein Auseinanderfallen des zusammengeschobenen Ringpakets.

Das Entnahmestück 1 besteht aus dem kalottenförmigen Bodenteil 12 und dem Entnahmestutzen 13. Am Bodenteil 12 sind einige, z.B. vier über den Umfang verteilte, elastisch verformbare Haken 14 angeformt, die in Ausnehmungen 15 im ersten Ring 8a eingreifen und damit Entnahmestück 1 und Gefäßkörper 3 verbinden.

Damit kann das Inhalationsgefäß nach der Erfindung für die Lagerung, den Versand, die Aufbewahrung und insbesondere auch auf Reisen, platzsparend verstaut und für den Gebrauch in einfachster Weise in die Gebrauchsstellung gemäß den Fig. 1 und 2 umgewandelt werden.

Zweckmäßig wird das Gefäß nach der Erfindung aus Kunststoffspritzteilen gefertigt.

## Ansprüche

1. Inhalationsgefäß bestehend aus einem Anschlußstück (1), einem Entnahmestück (2) und einem zwischen diesen liegenden Gefäßkörper (3), dadurch **gekennzeichnet,** daß der Gefäßkörper (3) aus ineinander verschiebbaren Ringen (8a,8b,8c ...8n) mit in Richtung auf das Anschlußstück (1) abnehmenden Durchmessern zusammengesetzt ist.

2. Inhalationsgefäß nach dem Anspruch 1, **dadurch gekennzeichnet,** daß jeder Ring (8a,8b, 8c...8n) mit einem dem Anschlußstück (1) zugewandten geringeren Durchmesser konisch ist.

3. Inhalationsgefäß nach dem Anspruch 2, **dadurch gekennzeichnet,** daß jeder Ring (8) über den Umfang verteilt zwei oder mehr Nuten (9) mit senkrechter Außenwand (10) aufweist, und die Nutenweite der einzelnen Ringe (8a,8b,8c...8n) von der Anschlußseite zur Entnahmeseite hin derart zunimmt, daß die jeweils obere (z.B. des Ringes 8c) in die jeweils untere Nut (z.B. des Ringes 8b) paßt.

4. Inhalationsgefäß nach dem Anspruch 1, **dadurch gekennzeichnet,** daß jeder Ring (8) auf der Seite seines größten Durchmessers einen Bund oder Innenflansch (11) aufweist.

5. Inhalationsgefäß nach dem Anspruch 1, **dadurch gekennzeichnet,** daß das Anschlußstück (1) aus einem Stutzen (4), einem sich daran anschließenden konischen Zwischenbereich (5) und einem zylindrischen Ringteil (6) besteht, welch letzterer in den obersten Ring (8n) geringsten Durchmessers eingreift.

6. Inhalationsgefäß nach dem Anspruch 1, **dadurch gekennzeichnet,** daß das Entnahmestück (2) aus einem Kalottenteil (12) und einem Entnahmestutzen (13) besteht.

7. Inhalationsgefäß nach dem Anspruch 6, **dadurch gekennzeichnet,** daß an dem Kalottenteil (12) des Entnahmeteils (2) über den Umfang verteilt elastisch verformbare Haken (14) angeformt sind, deren Enden in Ausnehmungen (15) des untersten Ringes (10) eingreifen.

FIG.1

FIG.2

FIG.4

FIG.3

FIG.5